# EUROPEAN PATENT SPECIFICATION

(11) **EP 0 552 267 B1**
(45) Date of publication and mention of the grant of the patent: **26.02.1997**
(21) Application number: 91919303.7
(22) Date of filing: 14.10.1991
(51) Int. Cl.: C12N 15/34, C12N 7/01, C12N 15/42, C12N 15/30, C12N 15/70, A61K 39/015, A61K 39/12

(54) **ENGINEERED BACTERIOPHAGES AND VACCINES CONTAINING THEM**
GENTECHNOLOGISCH VERÄNDERTE BAKTERIOPHAGEN UND SIE ENTHALTENDE IMPFSTOFFE
BACTERIOPHAGES MODIFIES PAR GENIE GENETIQUE ET VACCINS LES CONTENANT

(30) Priority: 12.10.1990 GB 9022190
(43) Date of publication of application: 28.07.1993
(73) Proprietor: PEPTIDE THERAPEUTICS LIMITED, Cambridge CB4 4NG (GB)
(72) Inventor: PERHAM, Richard, Nelson, Cambridge CB2 1QW (GB); WILLIS, Anne, Elizabeth, Cambridge CB2 1ND (GB); GREENWOOD, Judith, Impington, Cambridgeshire CB4 4LU (GB)
(74) Representative: Davies, Jonathan Mark
(86) International application number: GB9101785
(87) International publication number: WO9207077

(56) References cited:
- J. MOL. BIOL., vol. 220, August 1991, LONDON, UK, pages 821-827; J. GREENWOOD ET AL.
- JOURNAL OF BIOLOCAL CHEMISTRY, vol. 263, March 1988, BALTIMORE, US, pages 4318.4322; V. F. DE LA CRUZ ET AL.
- NATURE, vol. 330, 26 November 1987, LONDON, GB, pages 381-384; B. E. CLARKE ET AL.
- CHEMICAL ABSTRACTS, vol. 111, no. 19, 6 November 1989, Columbus, Ohio, US, abstract no. 171037; A. A. ILL'ICHEV ET AL.
- DOKL. AKAD. NAUK. SSSR, vol. 307, no. 2, 1989, MOSKOW, RUSSIA, pages 481-483.

## Description

### Field of the Invention

This invention relates to engineered bacteriophage and vaccines containing them.

### Background of the Invention

The treatment of infectious diseases remains a considerable problem. Major efforts are being made to develop new and/or improved vaccines. For a variety of reasons, progress is slow, but the discovery that protective immunity can be obtained by administration of only one of the constituent proteins of a pathogen, and in some cases only a fraction of protein, has offered new hope in the production of vaccines.

Foot-and-mouth disease virus (FMDV) belongs to the picornaviridae, a family of positive-sense RNA viruses. FMDV remains a major scourge of live-stock in several continents. The control of this disease is either by slaughter, in those countries where FMDV does not normally occur, or by vaccination in endemic areas. The vaccines that are currently in use are made from inactivated virus, which has been grown in tissue culture cells. This vaccine has proved to be very effective in controlling the disease, but there are many problems associated with handling large quantities of this highly infectious agent, and in ensuring its complete inactivation. Moreover, these vaccines are difficult to administer in remote regions, and severe economic losses due to the disease still occur.

Another good example of a widespread disease, and which has eluded imunologists for many years, is malaria. The major human malarial pathogen, Plasmodium falciparum, has a complex life-cycle and an ever-changing repertoire of surface antigens which allows highly effective evasion of the human immune-system. Numerous attempts have been made to control the disease but no satisfactory vaccine, suitable for widespread administration, has been obtained.

Irradiated sporozoites have been used to immunize man, and protection against malaria has been achieved. However, in this type of vaccine, both sexual and asexual forms of the parasite are required; sporozoites must, therefore, be prepared either from the salivary glands of infected mosquitoes or from cultured red blood cells. The former yields very small quantities, and the latter is a very expensive process.

The protective response to irradiated sporozoites has been localized to a protein (called the circumsporozoite protein) which is expressed on the surface of the organism and which does not mutate between generations. The most striking feature of this polypeptide chain is a large central repeat of the amino-acid sequence NANP (SEQ. ID No. 1), sometimes interspersed with the sequence NDVP (SEQ. ID No. 2). Further investigation has shown that twelve amino-acid units of this repeat sequence are sufficient for the production of antibodies reactive against the whole molecule. A wide variety of peptides, of twelve amino-acids or more, containing the repeat region of the circumsporozoite protein have been used to inoculate rabbits and mice; see, for example, Young et al.,Science 228 (1985) 985. Immunogenic responses have been obtained, but these are limited because oligopeptides do not generally provoke a humoral response.

Small peptides are poorly immunogenic, because they are missed by the cells of the immune-system or interact weakly with the cells they do meet. In addition, small peptides are excreted by the kidneys and, therefore, have only a short life-span in the blood-stream. In animal systems, some of these problems may be overcome by administering Freund's Complete Adjuvant (FCA) or aluminium hydroxide with the peptide, but both of these adjuvants are highly toxic and cannot be administered to humans. Peptides may be made more immunogenic if they are linked to a large carrier system after they have been synthesized, but this adds both to the complexity and expense of vaccine production.

To circumvent some of the problems associated with using small peptides as antigens, a different approach for vaccine production has been investigated. The genetic material of a virus is manipulated such that a foreign antigenic peptide is expressed on the surface of the virion. A DNA sequence which codes for the antigen is inserted into one of the coat protein genes; this may be a complex procedure, since many viruses contain RNA rather than DNA. For example, an antigenic epitope from polio virus type III has been inserted into the coat protein of tobacco mosaic virus; see Haynes et al., Biotechnology 4 (1986) 637. A hybrid coat protein was produced and over-expressed in E. coli and, after purification, it could be assembled into virus-type rods. The hybrid TMV particles, injected into rats, were able to stimulate the production of antibodies which could neutralize polio virus. Unfortunately, this response was only obtained when FCA was employed.

Antibodies have also been expressed on the surface of attenuated animal viruses such as vaccinia, herpes and polio; see Dimmock et al (ed.), Foc. Gen. Microbiol. Symp. 45, "Control of Virus Diseases", pub. Cambridge Univ. Press (1990). There are several problems associated with using animal viruses as vaccines: they are complex and their genetic material may be difficult to manipulate; they are difficult to prepare in large amounts and hence are expensive; they can sometimes revert to pathogenesis or exhibit some other form of toxicity.

Filamentous bacteriophages contain a single-stranded DNA genome which is encased within a protein sheath. The DNA becomes double-stranded after infection of the host strain, Escherichia coli, and both double- and single-stranded forms of DNA may be easily purified and manipulated (the virus M13, which is commonly used as a vector for site-directed mutagenesis and sequencing, is a filamentous bacteriophage). The genome encodes ten proteins, five of which are found in the mature phage particle. Gene VIII encodes the major coat protein which forms the tubular structure of the phage capsid, genes VII and IX encode minor coat proteins which are located at one end of the phage particle, and genes III and VI encode minor coat proteins positioned at the other end. A virus particle contains about five copies of each of the minor coat proteins.

The product of gene III is a large protein involved in infection of the host cell. Chemically-synthesized DNA cassettes have been cloned into gene III and recombinant proteins have been produced; see de la Cruz et al., J. Biol. Chem. 9 (1988) 4318. A number of recombinant proteins have been incorporated into viral particles and these were used to inoculate rabbits and mice, but in no case did the immune-system respond with sufficient vigour to confer immunity. Although phages engineered in this way do not seem to be suitable for use as vaccines (since large and potentially dangerous doses would be required to elicit an effective response), this system is being developed for production of "epitope-libraries" used, for example, to investigate the specificity of antibodies; see Scott & Smith, Science 249 (1990) 386.

Il'ichev et al, Doklady Akademii Nauk SSR 307(2) (July 1989) 481-3, disclose phage M13 having a foreign peptide, 5 amino-acids long, incorporated into the major envelope protein. A "substantial effect on the infectiousness of the phage" is reported; this effect is actually a considerable decrease, to about 10%, of the reinfection potential. No other properties of the engineered virus were reported.

Rowitsch et al, J. Mol. Biol. 204 (1988) 663-674, disclose hybrid phage assembly.

### Summary of the Invention

According to the present invention, a filamentous bacteriophage includes, in at least a proportion of its major coat protein, a foreign peptide of 6 or more amino-acids that is capable of eliciting a biological response.

The present invention is based on the realisation that the failure of the gene III fusion proteins in vaccine production is almost certainly due to the low number of proteins found in each phage and/or to the masking of the epitope by the bulk virus structure. The major coat protein (the product of gene VIII), however, is found as about 2500 copies per virus, and peptides having an immunogenic effect have now been inserted into this protein. Moreover, they are expressed in a region of the coat protein known to be exposed on the surface of the virus. As indicated below, the coat protein containing the foreign peptide may desirably be present in balance with unmodified (wild-type) virus coat protein.

### Description of the Drawings

Figure 1 is a schematic view of a hybrid filamentous bacteriophage. It shows a gene III protein 1, a coat protein with epitope 2, a wild-type coat protein 3, and an epitope 4 that is too large for normal packing of subunits.

Figure 2 shows the construction of plasmid pKfdH. The process involves isolating the NlaIV fragment and then the SnaBI/NlaIV fragment, followed by ligation into SmaI/phosphatase-treated pKK223-3.

### Description of the Invention

A bacteriophage of the invention includes a foreign peptide: the peptide may be antigenic, e.g. any that raises a desired immunological response, e.g. against a form of the malaria parasite or against another virus such as foot-and-mouth disease virus. It may already be known for this purpose. Its length should be sufficient to raise the response but insufficient to modify the bacteriophage's properties undesirably or to prevent its incorporation, e.g. at least 9 amino-acids.

A bacteriophage of the invention is preferably immunogenic, and is suitable for use in vaccines, and generally as a therapeutic/diagnostic product. Therefore, products of the invention may be formulated with any suitable physiologically-acceptable diluent or carrier, to prepare a vaccine composition.

In addition, by using two or more compatible vectors to carry suitably engineered coat protein genes, two or more differently modified coat proteins can be assembled into the same virion, to enable more than one peptide to be displayed simultaneously. This can lead to the generation of multi-shot vaccines and to the generation of more complex structures on the surface of the virion with correspondingly complex biological properties.

A potential disadvantage of this or other viruses as vaccines is that they contain DNA, which may be regarded as undesirable for injection into the animal being protected. With the filamentous bacteriophage carrier described here, the DNA within the virion may be selectively degraded by dialysis at acid pH before injection.

In an alternative embodiment of the invention, the foreign peptide is an agonist/antagonist of a cell receptor (e.g. a biologically-active peptide such as a hormone, releasing factor or growth factor) or an inhibitor of an enzyme (e.g. an inhibitor of renin or the AIDS virus proteinase) or an enzyme (phage enzyme) or an antibody (phage antibody) or any other protein or peptide with a desired biological activity. For some purposes, the peptide may include a non-metabolisable component. Bacteriophages including a variety of such peptides can be used to screen for preferred structure and activity. In addition, the hybrid phage system has other broad applications. By inserting varied or random DNA sequences to encode the peptides to be displayed, an infinite number of peptides can be expressed on the surface of the virion, thus creating "epitope libraries".

Epitope libraries allow large numbers (sometimes tens of millions) of small peptides to be surveyed for any desirable property, e.g. tight binding to an antibody, receptor or other binding protein. For example, while antibodies can be purified from the serum of a patient with an auto-immune disease, the target of the antibodies is often unknown. An epitope library can be used to find the antigenic target of the antibodies, hence identifying the physiological site of the disease and suggesting a route for therapeutic procedures. It may also be possible to discover peptides which mimic the biologically-active regions of hormones, cytokines, enzyme substrates, neuropeptides and other biomolecules. Peptides which could either replace or alter the action of the natural ligands would be powerful candidates for drug development. The hybrid phage allows the number of copies of a peptide displayed on each viral particle to be controlled within wide limits, and can confer great sensitivity.

The major coat protein of the filamentous bacteriophage fd is encoded by gene VIII. The protein is synthesized as a procoat which contains a 23 amino-acid leader-peptide attached to the N-terminus of the mature protein. After synthesis, the procoat protein is rapidly inserted into the inner-membrane of E. coli where it is processed to leave the 50 amino-acid mature coat protein spanning the membrane. This protein has three domains, a hydrophobic membrane-spanning domain, a positively-charged C-terminal domain which faces into the cytoplasm of the cell, and a negatively-charged N-terminal domain which extends into the periplasm. During assembly of virus articles, the coat protein subunits are pulled out of the cell membrane and become arranged in a helical array around the viral DNA. In the viral particle, the C-terminal region of the protein subunits faces inwards towards the DNA such that the positively-charged residues may neutralize the negative charges of the sugar-phosphate backbone. The N-terminal domain, by contrast, is on the outer surface of the particle where it is exposed to the environment. Nuclear magnetic resonance studies have indicated that this region is flexible both in the membrane-bound form of the protein and when presented on the outside of the phage particle. This region is also known to be highly antigenic, antibodies prepared against whole phage particles being directed almost solely towards this domain of the coat protein. In this invention, peptides are attached to the outside of the phage coat protein, to give extended coat proteins that successfully assemble into viral particles and elicit a strong immune-response.

Therefore, in order to prepare an embodiment of the invention, epitopes are inserted into the N-terminal domain of the major coat protein of bacteriophage fd, such that they decorate the outside of an assembled phage particle. This problem may be approached entirely at the DNA level, by engineering a restriction enzyme recognition site into gene VIII, and allowing insertion of oligonucleotide cassettes, encoding the desired epitope, directly into the phage genome. In order to study this problem in a controlled manner, however, it was preferred to separate the synthesis of any hybrid coat proteins from the assembly of phage particles. This allows the cloning and expression of novel coat proteins, and the membrane-insertion and packaging stages of phage assembly, to be studied as independent entities.

In order to allow independent expression of the fd coat protein gene, gene VIII may be subcloned into a controllable expression vector such as pKK223-3 which uses the tac promoter. However, the wild-type gene VIII does not contain a suitable restriction enzyme site for the insertion of oligonucleotide cassettes (which would be used to encode the epitopes). Therefore, before the gene is subcloned, a suitable site may be introduced by means of site-directed mutagenesis. By way of example, this site was blunt-ended (to allow "in-frame" insertion of DNA), was positioned between the third and fourth codons of the mature coat protein (in order to minimize disruption of the leader-peptidase recognition sequence in the procoat) and was recognised by HpaI which does not have any recognition sites in pKK223-3, thus providing a unique cloning site.

In Preparing a hybrid bacteriophage of the invention, an intermediate product is a plasmid encoding the modification. In order to prevent the loss of such a plasmid, it is preferred that the engineered coat protein is transplanted into a phage, phagemid or other replicon, with a packaging signal. The recombinant gene can then be packaged and selected for further replication.

The following Examples illustrate the preparation and utility of products of this invention. With the exception of the methods A, B and C described below, all experimental procedures are as described by Greenwood et al, J. Mol. Biol. 220 (1991) 821-827; see also Rowitsch et al (1988), supra.

### A Polyacrylamide gel electrophoresis using the tricine buffer system

Proteins were separated on denaturing polyacrylamide gels composed of: 49.5% (w/v) acrylamide/ 0.5% (w/v) bisacrylamide (7 ml), 3 M Tris HCl, pH 8.4 (7 ml), glycerol (4.2 ml) and H₂O (2.8 ml), set with 25% (w/v) ammonium persulphate (200 µ1) and TEMED (20 µ1). The stacking gel contained: 49.5% (w/v) acrylamide/ 0.5% (w/v) bisacrylamide (1 ml), 3 M Tris HCl, pH 8.4 (2.5 ml) and H₂O (6.8 ml), set with 25% (w/v) ammonium persulphate (100 µ1) and TEMED (10 µ1). The gel cassette measured 17 cm x 17 cm and was 1 cm thick.

Samples were loaded in 4 times their volume of denaturing buffer, i.e. DTT (154 mg), 10% (w/v) SDS (2 ml), glycerol (1 ml), 1 M Tris HCl, pH 6.8 (170 µl), H₂O (1.63 ml, 0.2 % (w/v) Bromophenol blue in ethanol (200 µl). Samples were heated at 100°C for 2 min before loading onto gels. The running buffer contained: 1.8% (w/v) Tricine, 1.2% (w/v) Tris base and 1% (w/v) SDS.

Gels were run overnight at 100 V or during the day at 250 V until the bromophenol blue dye front reached the bottom.

### B Electroblotting of proteins onto PVDF membrane

Proteins were separated using tricine-PAGE as described in Method A with the addition of 2 mM mercaptoacetic acid in the upper electrode buffer (used to scavenge N-blocking free radicals). When the gel had run to completion it was soaked in transfer buffer (25 mM Tris base, 190 mM glycine, 10% (v/v) MeOH) for 10 min. The PVDF membrane was prepared by immersion in MeOH for 10 s followed by equilibration in transfer buffer for 5 min. The proteins were electroblotted onto the PVDF membrane using a Biorad transblot apparatus. Transfer was carried out at 150 mA for 3-16 h with cooling to 16°C. When transfer was complete the PVDF was immediately soaked in water for 10 min with shaking. The membrane was rinsed in water and was allowed to air-dry thoroughly.

### C Detection of proteins on Western Blots using alkaline phosphatase

Proteins were electrophoretically transferred onto nitrocellulose. The membrane was soaked in TBST buffer (10 mM Tris HCl, pH 8.0, 0.9% (w/v) NaCl, 0.05% (v/v) Tween 20) for 10 min, followed by the same buffer containing 1% (w/v) BSA for 20 min. The membrane was washed with TBST (without BSA) for 10 min. The membrane was soaked in TBST containing antibody directed against bacteriophage fd or the hybrid bacteriophage (1 µl antibodies/1 ml TBST) for 30 min and was then washed in TBST 3 times (10 min per wash). After washing, goat anti-rabbit IgG-alkaline phosphatase-conjugate was added (1 µl antibodies/l ml TBST) and incubation was continued for 30 min. The membrane was washed 3 times as before and the blot was developed in AP buffer (100 mM Tris HCl, pH 9.0, 100 mM NaCl, 5 mM MgCl₂) containing about 1.6 mg nitro blue tetrazolium and about 0.8 mg 5-bromo-4-chloro-3-indolyl phosphate. Both of the substrates could be prepared as stocks in water shortly before use.

### Example 1

### 1.1 Site-directed mutagenesis and subcloning of fd gene VIII

An 18mer oligonucleotide (SEQ ID No. 3) was synthesized on a Biosearch 8600 machine using phosphoamidite chemistry, and was purified by polyacrylamide gel electrophoresis. Single-stranded DNA template was prepared from wild-type fd and mutagenesis was performed using the phosphorothioate method and available in kit form from Amersham International plc. A number of bacteriophages were selected and sequenced using a second oligonucleotide (SEQ ID No. 4, synthesized and prepared as above) which binds towards the 3'-end of gene VIII. A phage was isolated which bore the correct mutation and was designated fdH.

Insertion of the HpaI site led to a change in the amino-acid sequence of the mature coat protein (SEQ ID No. 5) from Gly-Asp at positions 3 and 4 to Val-Asn (SEQ ID No. 6). Such a change clearly did not significantly affect the membrane-insertion and processing of the procoat protein, nor the assembly of the coat protein into bacteriophage particles, since mutant phage were viable and could be propagated in yields comparable with the wild-type.

The scheme for subcloning the fdH gene VIII is shown in Figure 2. Double-stranded RF DNA of fdH was prepared and digested with the blunt-end cutter NlaIV to generate a number of fragments including a 292 bp fragment which contained gene VIII. This band was isolated by polyacrylamide gel electrophoresis and was further digested with a second blunt-end cutter, SnaBI, which removed 20 base pairs upstream of the gene (already known to interfere with efficient expression of the wild-type gene). The shortened fragment was again purified by polyacrylamide gel electrophoresis and was cloned into pKK223-3 which had been cut with SmaI and treated with calf intestine alkaline phosphatase. Recombinants which contained the fdH gene in the correct orientation for expression were identified by restriction digests and were designated pKfdH.

### 1.2 Expression of the fdH gene

Expression of the fdH gene was tested in vivo using E. coli strain TGl (lacIq) transformed with plasmid pKfdH. Transformed cells were grown in YT medium supplemented with 50 µg ampicillin/ml at 37°C to an A₆₀₀ value of approximately 0.3, and were then induced with IPTG (final concentration 250 µM). At various times after induction samples of cells were removed, collected by centrifugation, washed and analysed by means of polyacrylamide gel electrophoresis using the tricine buffer system (tricine-PAGE). Staining the gels with silver revealed that before induction there was no detectable coat protein produced; after induction, transformed cells contained increasing amounts of a protein which had an electrophoretic mobility similar to that of wild-type coat protein. In these in vivo experiments, the procoat protein is being inserted into the cell innermembrane and is being processed by leader-peptidase. In the case of the fdH protein, the mutations clearly had not interfered with either of these processes and there were large amounts of processed coat protein available in the cell membrane.

Therefore, the insertion of the HpaI site and the concomitant change in the primary structure of the procoat protein did not interfere with bacteriophage assembly. The mutated coat protein was successfully cloned into the controllable expression vector pKK223-3 where its synthesis could be induced at will. The procoat protein synthesized from the plasmid was also competent for membrane-insertion and was correctly processed.

Although it was perhaps unsurprising that the insertion of the HpaI site into fd gene VIII had little effect on the membrane-insertion and processing of the slightly altered procoat protein, the addition of a number of extra amino-acids into this small protein was a novel and more dramatic procedure, and the effects on membrane-insertion could not be predicted. Failure at this stage would obviously prohibit assembly of the fusion coat proteins into viral particles.

The second stage of the work, therefore, was to insert an oligonucleotide cassette into the HpaI site and to investigate the effects on the membrane-insertion and processing of the resulting procoat protein. The chosen epitope (SEQ ID No. 7) was the repeat sequence of the Plasmodium falciparum coat protein (SEQ ID No. 1). This was known to elicit an immune response when administered as a synthetic peptide and, at only 12 amino-acids, was one of the smallest well-defined epitopes.

### 1.3 Insertion of DNA cassettes into the fdH gene

Two oligonucleotides were synthesized and purified as described in Section 1.1. One was SEQ ID No. 7, encoding (NANP)₃, and the other was the complementary sequence (SEQ ID No. 8). To produce the double-stranded DNA cassette, equal amounts of these two oligonucleotides were heated to 67°C and allowed to cool slowly to room temperature. The 36 bp cassette was ligated into pKfdH DNA which had been treated with HpaI and calf intestine alkaline phosphatase. Ligated plasmids were transformed into E. coli strain JM109 (lacIq, rec 0). Recombinants could first be identified by restriction analysis since successful insertion of DNA would destroy the HpaI site. Plasmids which were found to be resistant to digestion with HpaI were sequenced in order to determine the number and orientation of inserted cassettes. A clone was isolated that contained one copy of the 36 bp cassette in the correct orientation for expression and was designated pKfdmal.

### 1.4 Expression of the fd fusion protein fdMal in vivo

E. coli cells (strain JM109) harbouring plasmid pKfdMal are induced with IPTG exactly as described for cells harbouring pKfdH (Section 1.2). Before induction only E. coli proteins were present; after induction, increasing amounts of a protein appeared which was considerably larger than the wild-type fd coat protein. Estimation of the molecular weight of the protein from polyacrylamide gel electrophoresis (approx. 7 kDa) was consistent with it being the fdMal fusion protein. Moreover, this result strongly suggested that the procoat fusion protein had been inserted into the cell membrane and correctly processed. Western-blotting of proteins from the induced cells revealed that the fusion protein was not recognised by antibodies raised against wild-type fd phage.

As a result, a general scheme for the production of fusion proteins in E. coli has been created; a gene containing an additional 36 base-pairs has been successfully cloned and expressed. The fusion protein contains twelve amino-acids inserted into the centre of the procoat protein (a 16% increase in size of the mature region), but these extra residues did not interfere with membrane-insertion of the protein. The leader-peptide was efficiently removed from membrane-bound procoat, indicating that the inserted epitope did not disrupt the leader-peptidase recognition sequence. Antibodies raised against wild-type fd phage did not recognise the fusion protein, suggesting that this system allows immunogenic specificity.

It is known from previous work that fd coat protein expressed from a plasmid in a non-suppressor strain of E. coli can support the growth of a bacteriophage, R252, that has an amber mutation in its own coat protein gene. In addition, it has been possible to produce 'hybrid' bacteriophage containing both wild-type coat proteins expressed from the phage genome and mutant coat proteins expressed from a plasmid within the host cell. This was possible even when the mutant coat protein could not support phage growth in the absence of wild-type coat protein. It has, therefore, been possible to study the ability of the fdMal coat protein to assemble into bacteriophage particles both in isolation and when 'diluted' with wild-type coat proteins.

### 1.5 Production of hybrid bacteriophages

E. coli strain JM109 (lacIq, su) transformed with plasmid pKfdMal was grown at 37°C with shaking in YT medium containing ampicillin (50 µg/ml) until a A₆₀₀ value of -0.3 was reached. The cells were then infected with either phage fd or phage R252, growth was continued for 20 min and cells were induced with IPTG (10 µM final concentration). Growth was allowed to continue overnight. Phage were harvested and purified as described by Greenwood et al (1991) supra, and samples were analysed by tricine-PAGE followed by staining with silver.

The yield of R252 phage was very poor and electrophoretic analysis revealed that the only coat protein present was of the same mobility as wild-type coat protein. The small amount of phage produced was therefore assumed to be due to the low level of reversion and pseudoreversion that occurs within the population of amber mutant phages.

The yield of fd phage, on the other hand, was good (approx. 10 mg purified phage from 1 l of culture). Electrophoretic analysis revealed the presence of two coat proteins, one with the same mobility as wild-type and one with the same mobility as the fdMal protein expressed from pKfdMal. These two proteins were present in almost equal amounts (there is only slightly more of the wild-type coat protein).

### 1.6 Characterisation of the fdMal protein

For absolute confirmation of the identity of the larger protein, a sample was separated from the wild-type protein by electrophoresis and was transferred onto PVDF membrane (method B). The N-terminal sequence was determined as SEQ ID No. 9, i.e. AEV(NANP)₃N. This indicated that the complete peptide had been inserted into the expected position in the coat protein and that normal processing of the procoat had occurred.

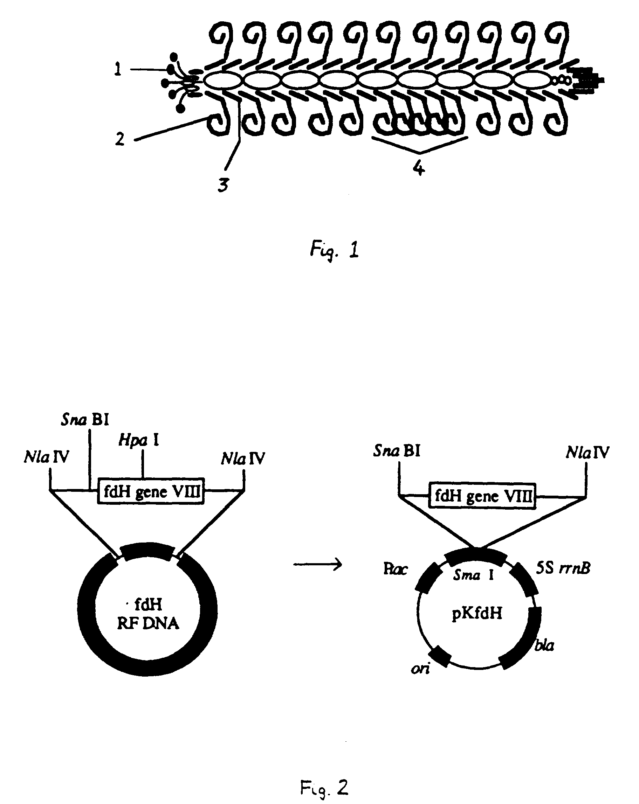

It was therefore apparent that the addition of twelve amino-acids into the N-terminal domain of the mature coat protein of the phage was incompatible with phage assembly unless the wild-type coat protein was also present. The most likely explanation for this is that the extra peptide is too bulky to allow acceptable packing of the coat proteins where the fusion proteins alone constitute the coat; however, when wild-type coat proteins are also present, these may act as 'spacers' allowing the fusion proteins to be incorporated. This has been represented diagrammatically in Figure 1. Although this adds slightly to the complexity of making phage containing fusion proteins (since it would clearly be futile to try to insert the oligonucleotide cassette directly into the phage genome), it may, in fact, be an advantage for the production of antibodies or other biologically-active peptide inserts, since the peptide is allowed more space to fold into the correct 3-dimensional structure, facilitating the production of an immune or other specific biological response.

There a number of important criteria for the successful use of the fdMal hybrid phages in vaccines. For example, it is necessary for a good immune response to be achieved without the addition of stimulants, such as Freund's Adjuvant or aluminium hydroxide, if the phage are to be injected into humans. It is important that the malaria epitope within the coat protein be available to the immune-system and that any antibodies produced against the fusion protein can also recognise the epitope when presented in a different form, such as the free peptide in solution (and eventually as part of the malaria parasite). Finally, it is important that the antibodies react specifically with the malaria epitope.

### 1.7 Production of antibodies

New Zealand White rabbits were injected subcutaneously with 0.5 mg samples, at 1 mg/ml, of either wild-type bacteriophage fd or wild-type/fdMal hybrid bacteriophages. Rabbits were injected on days 1, 14, 28, 38, 42, 56 and 66. No other compounds were administered. After 6 weeks the rabbits were bled out and the serum was isolated.

### 1.8 Western blot analysis

Samples of wild-type phage and wild-type/fdMal hybrid phage were subjected to tricine-PAGE and were electrophoretically transferred to nitrocellulose. Western blots were prepared using either anti-fd or anti-fd/fdMal antibodies. The anti-fd antibodies strongly recognised the wild-type fd cost protein but did not recognise the fdMal protein. Anti-fd/fdMal antibodies recognised both the fd protein and the fdMal protein. This was expected since both proteins are present in the hybrid phage which were injected into rabbits. However, the reaction against the fdMal protein was noticeably stronger than the reaction against the wild-type protein. This suggested two things: (1) that the larger fusion proteins were either more antigenic than the fd coat proteins or that they were shielding the wild-type proteins from the immune-system, so that there were more antibodies produced against fdMal than against fd; (2) that the anti-fdMal antibodies were not recognising the wild-type coat proteins. Both of these factors suggested that the hybrid phage had generated a response predominantly directed against the malaria epitope attached to the phage rather than the phage themselves.

### 1.9 Recognition of synthetic peptides

The antibodies generated against the fd/fdMal hybrid phage clearly reacted against the fdMal fusion protein; however, it was important that it could also recognise the malaria epitope when it was presented in a different form. The easiest way of testing for this was to check reactivity against an (NANP)₃ peptide that had been chemically synthesized.

In fact, synthesis of (NANP)₃ itself is technically difficult because the C-terminal proline residue causes premature termination. To circumvent these difficulties and extra amino-acid (asn = N) was added to the C-terminal end. Samples of purified (NANP)₃N (see SEQ ID No. 10) were spotted onto nitrocellulose and were allowed to dry. For comparison, a second unrelated peptide (SEQ ID No. 11), a sample of phage fd and a sample of phage fd/fdMal were treated in a similar fashion. These samples were incubated with either anti-fd antibody (1:1000 dilution) or anti-fd/fdMal antibody (at dilutions of 1:500, 1:1000, 1:2000, 1:5000). After washing the filters, goat anti-rabbit IgG-alkaline phosphatase-conjugate was added. Following further washing, the filters were developed by addition of nitro blue tetrazolium and 5-bromo-4-chloro-3-indolyl phosphate.

The antibodies raised against bacteriophage fd reacted with both the fd and fd/fdMal hybrid phage samples. These antibodies did not cross-react at all with either the synthetic (NANP)₃N peptide or with the control peptide. Antibodies raised against fd/fdMal hybrids, however, reacted not only with both pure and hybrid phage samples, but also very strongly with the synthetic (NANP)₃N peptide. No reaction was seen with the control peptide.

Injection of hybrid bacteriophage into rabbits, without the addition of an immunostimulant, such as Freund's Complete Adjuvant, was sufficient to generate a strong immune-response. A high titre of antibodies was produced and these reacted strongly with a sample of hybrid bacteriophage. The mixed population of antibodies had a great affinity for the malaria fusion protein, could recognise a synthetic version of the malaria peptide and did not cross-react with an unrelated peptide. Hence, the immune-response was both vigorous and very specific.

In summary of Example 1, a malaria parasite peptide has been inserted into the protein capsid of bacteriophage fd; the vigorous production of antibodies in rabbits and the ability of the antibodies to cross-react strongly with a synthetic malaria peptide have been demonstrated.

### Example 2

In this Example, oligonucleotides were designed and subcloned into gene VIII DNA to enable a region of the 141-160 epitope from FMDV Vp1 to be displayed on the outside of the phage particles.

### 2.1 Making fd/fd FMDV hybrid bacteriophage

Oligonucleotide cassettes (SEQ ID Nos. 12 and 13) were synthesised. These encode the region 141-154 in FMDV Vp1. The oligonucleotides were treated with the enzyme polynucleotide kinase, annealed and ligated into pKfdH (as previously described). Plasmids containing the cassettes in the correct orientation were screened for by DNA sequencing. Hybrid bacteriophage were made (as described above) which contained a ratio of wild-type to extended coat protein of 3:1. Rabbits were inoculated with these bacteriophage; serum was isolated and assayed using an ELISA system. Either bacteriophage at 0.1 µg/ml or chemically-synthesised peptide SEQ ID No. 14 cross-linked to keyhole limpet haemacyanin was adsorbed onto 96-well plates in 0.1M sodium carbonate buffer, pH 8.3. After 16 hours at room temperature the unoccupied sites were blocked by the addition of 5% bovine serum albumin in Tris-buffered saline and 0.1% Tween 20 (TBST) for 2 hours at room temperature. The rabbit serum was then added to the plates at a range of dilutions.

After 30 min at room temperature, the serum was removed and the plates washed with TBST. The plates were incubated for 30 min with goat anti-rabbit IgG cross-linked to alkaline phosphatase at 1:1000 dilution. The plates were washed to remove excess secondary antibody and the presence of the alkaline phosphatase located using an AMPAK system (Novo Bio).

The rabbit serum was found to react strongly with both the hybrid bacteriophage and the chemically-synthesised peptide, showing that the rabbit was responding to the epitopes displayed on the outside of the bacteriophage.

### 2.2 Testing the fd/FMDV bacteriophage as a vaccine

Guinea pigs (4 in each of 5 groups plus control animals) were inoculated with the fd/FMDV hybrid bacteriophage and various adjuvants. The animals were then challenged with live virus.

The amounts of phage, the adjuvants and the results are given in the following Table (FICA = Freund's Incomplete Adjuvant). The results clearly illustrate that the fd/fd FMDV hybrid bacteriophage provide protection against FMDV.

| Dose | Adjuvant | Protection | Lesion |
|---|---|---|---|
| 0 | - | 0/4 | 12 |
| 500 µg | PBS | 2/4 | 2 |
| 500 µg | FICA | 4/4 | 0 |
| 500 µg | Al(OH)₃ | 4/4 | 0 |
| 50 µg | FICA | 2/4 | 5 |
| 50 µg | Al(OH)₃ | 1/4 | 8 |

In summary, Example 2 demonstrates that, by displaying epitopes on the surface of bacteriophage, it is possible to create an highly effective vaccine to FMDV. This vaccine has many powerful advantages over those that are already commercially available. It is very stable and does not require refrigeration. It is extremely cheap and easy to produce. Moreover, a wide range of different epitopes can be displayed on the surface of the bacteriophage to create a vaccine that can protect against number of serotypes. This technology can be used to develop vaccines against numerous infectious diseases including AIDS.

### Example 3

A unique HpaI-restriction site was engineered between the codons specifying residues 3 and 4 of the mature coat protein (to generate vector fdH), and an oligonucleotide encoding the hexapeptide YGFWGM (SEQ. ID No. 15) was cloned at this site into the bacteriophage fdH RF DNA. Viable progeny were isolated in all cases. On SDS-polyacrylamide gel electrophoresis, the lowered mobility of the enlarged coat protein was consistent with the insertion of an additional 6-amino-acids, and its amino-acid composition was found to be similarly consistent. However, it did not prove possible to insert more than 6 amino-acids directly into the major coat protein of bacteriophage fd by this technique. This might be due to one or more of several reasons: instability of the modified protein, a failure of membrane-insertion and processing of the novel procoat molecule, or an inability subsequently to package the DNA.

### SEOUENCE LISTING

SEQ ID No. 1
   Sequence Type: Peptide
   Sequence Length: 4 amino-acids
   Original Source Organism: Plasmodium falciparum Asn Ala Asn Pro
SEQ ID No. 2
   Sequence Type: Peptide
   Sequence Length: 4 amino-acids
   Original Source Organism: Plasmodium falciparum Asn Asp Val Pro
SEQ ID No. 3
   Sequence Type: Nucleotide
   Sequence Length: 18 bases
   Strandedness: Single
   Topology: Linear GGGATCGTTA ACCTCAGC
SEQ ID No. 4
   Sequence Type: Nucleotide
   Sequence Length: 17 bases
   Strandedness: Single
   Topology: Linear CCATCGCCCA CGCATAA
SEQ ID No. 5
   Sequence Type: Peptide
   Sequence Length: 9 amino-acids
   Original Source Organism: Bacteriophage fdh
   Feature: Ala Ala Leader-Peptidase recognition site 
Ser Phe Ala Ala Glu Gly Asp Asp Pro
        -1  1               5
SEQ ID No. 6
   Sequence Type: Peptide
   Sequence Length: 9 amino-acids
   Original Source Organism: Bacteriophage fdh
   Feature: Val Asn Insertion site 
Ser Phe Ala Ala Glu Val Asn Asp Pro
        -1  1               5
SEQ ID No. 7
   Sequence Type: Nucleotide with corresponding peptide
   Sequence Length: 36 bases
   Strandedness: Single
   Topology: Linear 
AAT GCA AAC CCG AAC GCA AAC CCG AAT GCA AAC CCG
Asn Ala Asn Pro Asn Ala Asn Pro Asn Ala Asn Pro
                5                   10
SEQ ID No. 8
   Sequence Type: Nucleotide
   Sequence Length: 36 bases
   Strandedness: Single
   Topology: Linear CGG GTT TGC ATT CGG GTT TGC GTT CGG GTT TGC ATT
SEQ ID No. 9
   Sequence Type: Peptide N-terminal
   Sequence Length: 16 amino-acids 
Ala Glu Val Asn Ala Asn Pro Asn Ala Asn Pro Asn Ala Asn Pro Asn
                5                   10                  15
SEQ ID No. 10
   Sequence Type: Peptide
   Sequence Length: 13 amino-acids 
Asn Ala Asn Pro Asn Ala Asn Pro Asn Ala Asn Pro Asn
                5                   10
SEQ ID No. 11
   Sequence Type: Peptide
   Sequence Length: 11 amino-acids 
Ala Ala Pro Ala Ala Ala Pro Ala Pro Ala Ala
                5                   10
SEQ ID No. 12
   Sequence Type: Nucleotide
   Sequence Length: 36 bases
   Strandedness: Single
   Topology: Linear CGG CAG GGT GCG CGC CAC TTT CTG CGC CAG CAC CTG
SEQ ID No. 13
   Sequence Type: Nucleotide
   Sequence Length: 36 bases
   Strandedness: Single
   Topology: Linear CTG CGC CAG CAC CTG CAG ATC GCC GCG CAG GTT CGG
SEQ ID No. 14
   Sequence Type: Peptide
   Sequence Length: 19 amino-acids 
Val Pro Asn Leu Arg Gly Asp Gln Val Leu Ala Gln Lys Val Ala
                5                   10                  15
Arg Thr Leu Pro
SEQ ID No. 15
   Sequence Type: Nucleotide with corresponding peptide
   Sequence Length: 18 bases
   Original Source Organism: N-terminal peptide of β-endorphin

TAT GGT TTC TGG GGT ATG
Tyr Gly Phe Trp Gly Met
                5

## Claims (Claims for the following Contracting State(s): AT, BE, CH, DE, DK, FR, GB, IT, LI, LU, NL, SE)

1. A filamentous bacteriophage including, in at least a proportion of its major coat protein sub-units, multiple display of a foreign peptide of a least 6 amino-acids that elicits a biological response.

2. A bacteriophage according to claim 1, wherein the peptide has at least 9 amino-acids.

3. A bacteriophage according to claim 1 or claim 2, wherein two or more different peptides are displayed simultaneously.

4. A bacteriophage according to any of claims 1 to 3, wherein the or each peptides is an agonist or antagonist of a cell receptor or an enzyme inhibitor.

5. A bacteriophage according to any of claims 1 to 3, wherein the or each peptide is antigenic or immunogenic.

6. A bacteriophage according to claim 5, wherein the or each peptide raises a response against malaria.

7. A bacteriophage according to claim 5, wherein the or each peptide raises a response against foot-and-mouth disease.

8. A bacteriophage according to any preceding claim, whose DNA is degraded.

9. A vaccine composition comprising a bacteriophage according to any of claims 5 to 8, in association with a physiologically-acceptable carrier or diluent.

10. A method for preparing a bacteriophage according to any of claims 1 to 7, which comprises introducing a unique restriction enzyme site into gene VIII, subcloning the thus modified gene VIII into a controllable expression vector, inserting one or more cassettes encoding the or each peptide into the vector, and assembling the protein product of the resultant vector into the wild-type bacteriophage.

11. A method according to claim 10, wherein the gene encoding the engineered coat protein is transplanted into a replicon including a packaging signal.

## Claims (Claims for the following Contracting State(s): ES, GR)

1. A filamentous bacteriophage including, in at least a proportion of its major coat protein sub-units, multiple display of a foreign peptide of a least 6 amino-acids that elicits a biological response.

2. A bacteriophage according to claim 1, wherein the peptide has at least 9 amino-acids.

3. A bacteriophage according to claim 1 or claim 2, wherein two or more different peptides are displayed simultaneously.

4. A bacteriophage according to any of claims 1 to 3, wherein the or each peptides is an agonist or antagonist of a cell receptor or an enzyme inhibitor.

5. A bacteriophage according to any of claims 1 to 3, wherein the or each peptide is antigenic or immunogenic.

6. A bacteriophage according to claim 5, wherein the or each peptide raises a response against malaria.

7. A bacteriophage according to claim 5, wherein the or each peptide raises a response against foot-and-mouth disease.

8. A bacteriophage according to any preceding claim, whose DNA is degraded.

9. A vaccine composition comprising a bacteriophage according to any of claims 5 to 8, in association with a physiologically-acceptable carrier or diluent.

10. A method for preparing a bacteriophage according to any of claims 1 to 7, which comprises introducing a unique restriction enzyme site into gene VIII, subcloning the thus modified gene VIII into a controllable expression vector, inserting one or more cassettes encoding the or each peptide into the vector, and assembling the protein product of the resultant vector into the wild-type bacteriophage.

11. A method according to claim 10, wherein the gene encoding the engineered coat protein is transplanted into a replicon including a packaging signal.

12. A method for producing an engineered bacteriophage, which comprises assembling a filamentous bacteriophage including, in at least a proportion of its major coat protein sub-units, multiple display of a foreign peptide of at least 6 amino-acids that elicits a biological response.

13. A method according to claim 12, wherein the peptide has at least 9 amino-acids.

14. A method according to claim 12 or claim 13, wherein two or more different peptides are displayed simultaneously.

15. A method according to any of claims 12 to 14, wherein the or each peptide is an agonist or antagonist of a cell receptor or an enzyme inhibitor.

16. A method according to any of claims 12 to 14, wherein the or each peptide is antigenic or immunogenic.

17. A method according to claim 16, wherein the or each peptide raises a response against malaria.

18. A method according to claim 16, wherein the or each peptide raises a response against foot-and-mouth disease.

19. A method according to any preceding claim, wherein the DNA of the bacteriophage is degraded.

20. A method for producing a vaccine composition comprising associating a bacteriophage made according to any of claims 16 to 19 with a physiologically-acceptable carrier or diluent.

## Patentansprüche (Patentansprüche für folgende(n) Vertragsstaat(en): AT, BE, CH, DE, DK, FR, GB, IT, LI, LU, NL, SE)

1. Fadenartiger Bakteriophage, enthaltend, wenigstens in einem Anteil seiner Haupt-Hüllproteinuntereinheiten, eine Mehrfach-Darstellung eines Fremdpeptids von mindestens 6 Aminosäuren, das eine biologische Reaktion auslöst.

2. Bakteriophage nach Anspruch 1, wobei das Peptid mindestens 9 Amino-Säuren hat.

3. Bakteriophage nach Anspruch 1 oder Anspruch 2, wobei zwei oder mehr verschiedene Peptide gleichzeitig dargestellt sind.

4. Bakteriophage nach einem der Ansprüche 1 bis 3, wobei das oder jedes Peptid ein Agonist oder ein Antagonist eines Zellerezeptors oder eines Enzymhemmstoffs ist.

5. Bakteriophage nach einem der Ansprüche 1 bis 3, wobei das oder jedes Peptid antigen oder immunogen ist.

6. Bakteriophage nach Anspruch 5, wobei das oder jedes Peptid eine Reaktion gegen Malaria hervorruft.

7. Bakteriophage nach Anspruch 5, wobei das oder jedes Peptid eine Reaktion gegen Maul- und Klauenseuche hervorruft.

8. Bakteriophage nach einem der vorhergehenden Ansprüche, dessen DNS degradiert ist.

9. Impfstoffverbindung, enthaltend einen Bakteriophagen nach einem der Ansprüche 5 bis 8 in Assoziation mit einem physiologisch verträglichen Träger oder Verdünnungsmittel.

10. Verfahren zur Herstellung eines Bakteriophagen nach einem der Ansprüche 1 bis 7, enthaltend Einführen einer eindeutigen Restriktionsenzymstelle in Gen VIII, Subklonen des auf diese Art modifizierten Gens VIII in einen kontrollierbaren Expressionsvektor, Einfügen einer oder mehrerer, das oder jedes Peptid kodierender Kassetten in den Vektor und Einbauen des Proteinprodukts des resultierenden Vektors in den Wildtyp-Bakteriophagen.

11. Verfahren nach Anspruch 10, wobei das das gen-veränderte Hüllprotein kodierende Gen in ein Replikon transplantiert wird, das ein Packsignal enthält.

## Patentansprüche (Patentansprüche für folgende(n) Vertragsstaat(en): ES, GR)

1. Fadenartiger Bakteriophage, enthaltend, wenigstens in einem Anteil seiner Haupt-Hüllproteinuntereinheiten, eine Mehrfach-Darstellung eines Fremdpeptids von mindestens 6 Aminosäuren, das eine biologische Reaktion auslöst.

2. Bakteriophage nach Anspruch 1, wobei das Peptid mindestens 9 Amino-Säuren hat.

3. Bakteriophage nach Anspruch 1 oder Anspruch 2, wobei zwei oder mehr verschiedene Peptide gleichzeitig dargestellt sind.

4. Bakteriophage nach einem der Ansprüche 1 bis 3, wobei das oder jedes Peptid ein Agonist oder ein Antagonist eines Zellrezeptors oder eines Enzymhemmstoffs ist.

5. Bakteriophage nach einem der Ansprüche 1 bis 3, wobei das oder jedes Peptid antigen oder iminunogen ist.

6. Bakteriophage nach Anspruch 5, wobei das oder jedes Peptid eine Reaktion gegen Malaria hervorruft.

7. Bakteriophage nach Anspruch 5, wobei das oder jedes Peptid eine Reaktion gegen Maul- und Klauenseuche hervorruft.

8. Bakteriophage nach einem der vorhergehenden Ansprüche, dessen DNS degradiert ist.

9. Impfstoffverbindung, enthaltend einen Bakteriophagen nach einem der Ansprüche 5 bis 8 in Assoziation mit einem physiologisch verträglichen Träger oder Verdünnungsmittel.

10. Verfahren zur Herstellung eines Bakteriophagen nach einem der Ansprüche 1 bis 7, enthaltend Einführen einer eindeutigen Restriktionsenzynstelle in Gen VIII, Subklonieren des auf diese Art modifizierten Gens VIII in einen kontrollierbaren Expressionsvektor, Insertion einer oder mehrerer, das oder jedes Peptid kodierender Kassetten in den Vektor und Einbauen des Proteinprodukts des resultierenden Vektors in den Wildtyp-Bakteriophagen.

11. Verfahren nach Anspruch 10, wobei das das gen-veränderte Hüllprotein kodierende Gen in ein Replikon transplantiert wird, das ein Packsignal enthält.

12. Verfahren zur Herstellung eines gentechnologisch veränderten Bakteriophagen, enthaltend den Zusammenbau eines fadenartigen Bakteriophagen, der wenigstens in einem Anteil seiner Haupt-Hüllproteinuntereinheiten eine Mehrfach-Darstellung eines Frerndpeptids von mindestens 6 Aminosäuren, das eine biologische Reaktion auslöst, enthält.

13. Verfahren nach Anspruch 12, wobei das Peptid mindestens 9 Amino-Säuren hat.

14. Verfahren nach Anspruch 12 oder Anspruch 13, wobei zwei oder mehr verschiedene Peptide gleichzeitig dargestellt werden.

15. Verfahren nach einem der Ansprüche 12 bis 14, wobei das oder jedes Peptid ein Agonist oder ein Antagonist eines Zellrezeptors oder eines Enzymhemmstoffs ist.

16. Verfahren nach einem der Ansprüche 12 bis 14, wobei das oder jedes Peptid antigen oder immunogen ist.

17. Verfahren nach Anspruch 16, wobei das oder jedes Peptid eine Reaktion gegen Malaria hervorruft.

18. Verfahren nach Anspruch 16, wobei das oder jedes Peptid eine Reaktion gegen Maul- und Klauenseuche hervorruft.

19. Verfahren nach einem der vorhergehenden Ansprüche, wobei die DNS des Bakteriophagen degradiert wird.

20. Verfahren zur Herstellung einer Impfstoffzusammensetzung, enthaltend die Assoziation eines nach einem der Ansprüche 16 bis 19 hergestellten Bakteriophagen mit einem physiologisch verträglichen Träger oder Verdünnungsmittel.

## Revendications (Revendications pour l'(les) Etat(s) contractant(s) suivant(s): AT, BE, CH, DE, DK, FR, GB, IT, LI, LU, NL, SE)

1. Bactériophage filamenteux incluant, dans au moins une certaine proportion de ses sous-unités majeures de protéines d'enveloppe, la présentation multiple d'un peptide étranger d'au moins 6 acides aminés qui déclenche une réponse biologique.

2. Bactériophage selon la revendication 1, dans lequel le peptide a au moins 9 acides aminés.

3. Bactériophage selon la revendication 1 ou la revendication 2, dans lequel deux ou plusieurs peptides différents sont présentés simultanément.

4. Bactériophage selon l'une quelconque des revendications 1 à 3, dans lequel le ou chaque peptide est un agoniste ou antagoniste d'un récepteur cellulaire ou d'un inhibiteur enzymatique.

5. Bactériophage selon l'une quelconque des revendications 1 à 3, dans lequel le ou chaque peptide est antigénique ou immunogène.

6. Bactériophage selon la revendication 5, dans lequel le ou chaque peptide suscite une réponse contre le paludisme.

7. Bactériophage selon la revendication 5, dans lequel le ou chaque peptide suscite une réponse contre la fièvre aphteuse.

8. Bactériophage selon l'une quelconque des revendications précédentes, dont l'ADN est dégradé.

9. Composition de vaccin comprenant un bactériophage selon l'une quelconque des revendications 5 à 8, en association avec un support ou diluant physiologiquement acceptable.

10. Procédé de préparation d'un bactériophage selon l'une quelconque des revendications 1 à 7, dans lequel on introduit un site d'enzyme de restriction unique dans le gène VIII, on sous-clone le gène VIII ainsi modifié dans un vecteur d'expression régulable, on insère une ou plusieurs cassettes codant pour le ou chaque peptide dans le vecteur, et on assemble le produit protéique du vecteur résultant dans le bactériophage de type sauvage.

11. Procédé selon la revendication 10, dans lequel on transplante le gène codant pour la protéine d'enveloppe produite par génie génétique dans un réplicon incluant un signal d'encapsidation.

## Revendications (Revendications pour l'(les) Etat(s) contractant(s) suivant(s): ES, GR)

1. Bactériophage filamenteux incluant, dans au moins une certaine proportion de ses sous-unités majeures de protéines d'enveloppe, la présentation multiple d'un peptide étranger d'au moins 6 acides aminés qui déclenche une réponse biologique.

2. Bactériophage selon la revendication 1, dans lequel le peptide a au moins 9 acides aminés.

3. Bactériophage selon la revendication 1 ou la revendication 2, dans lequel deux ou plusieurs peptides différents sont présentés simultanément.

4. Bactériophage selon l'une quelconque des revendications 1 à 3, dans lequel le ou chaque peptide est un agoniste ou antagoniste d'un récepteur cellulaire ou d'un inhibiteur enzymatique.

5. Bactériophage selon l'une quelconque des revendications 1 à 3, dans lequel le ou chaque peptide est antigénique ou immunogène.

6. Bactériophage selon la revendication 5, dans lequel le ou chaque peptide suscite une réponse contre le paludisme.

7. Bactériophage selon la revendication 5, dans lequel le ou chaque peptide suscite une réponse contre la fièvre aphteuse.

8. Bactériophage selon l'une quelconque des revendications précédentes, dont l'ADN est dégradé.

9. Composition de vaccin comprenant un bactériophage selon l'une quelconque des revendications 5 à 8, en association avec un support ou diluant physiologiquement acceptable.

10. Procédé de préparation d'un bactériophage selon l'une quelconque des revendications 1 à 7, dans lequel on introduit un site d'enzyme de restriction unique dans le gène VIII, on sous-clone le gène VIII ainsi modifié dans un vecteur d'expression régulable, on insère une ou plusieurs cassettes codant pour le ou chaque peptide dans le vecteur, et on assemble le produit protéique du vecteur résultant dans le bactériophage de type sauvage.

11. Procédé selon la revendication 10, dans lequel on transplante le gène codant pour la protéine d'enveloppe produite par génie génétique dans un réplicon incluant un signal d'encapsidation.

12. Procédé de production d'un bactériophage par génie génétique, dans lequel on assemble un bactériophage filamenteux incluant, dans au moins une certaine proportion de ses sous-unités majeures de protéine d'enveloppe, une présentation multiple d'un peptide étranger d'au moins 6 acides aminés qui déclenche une réponse biologique.

13. Procédé selon la revendication 12, dans lequel le peptide a au moins 9 acides aminés.

14. Procédé selon la revendication 12 ou la revendication 13, dans lequel deux ou plusieurs peptides différents sont présentés simultanément.

15. Procédé selon l'une quelconque des revendications 12 à 14, dans lequel le ou chaque peptide est un agoniste ou antagoniste d'un récepteur cellulaire ou d'un inhibiteur enzymatique.

16. Procédé selon l'une quelconque des revendications 12 à 14, dans lequel le ou chaque peptide est antigénique ou immunogène.

17. Procédé selon la revendication 16, dans lequel le ou chaque peptide suscite une réponse contre le paludisme.

18. Procédé selon la revendication 16, dans lequel le ou chaque peptide suscite une réponse contre la fièvre aphteuse.

19. Procédé selon l'une quelconque des revendications précédentes, dans lequel l'ADN du bactériophage est dégradé.

20. Procédé de production d'une composition de vaccin comprenant l'association d'un bactériophage produit selon l'une quelconque des revendications 16 à 19 avec un support ou diluant physiologiquement acceptable.
